# EUROPEAN PATENT SPECIFICATION

(11) **EP 4 362 899 B1**
(45) Date of publication and mention of the grant of the patent: **27.11.2024**
(21) Application number: 22733606.2
(22) Date of filing: 13.06.2022
(51) Int. Cl.: A61K 8/39, A61K 8/73, A61Q 5/12, A61K 8/55

(54) **TRANSPARENT AND LOW FOAMING COSMETIC AND PERSONAL CARE COMPOSITIONS**
TRANSPARENTE UND WENIG SCHÄUMENDE KOSMETIK- UND KÖRPERPFLEGEZUSAMMENSETZUNGEN
COMPOSITIONS COSMÉTIQUES ET DE SOINS PERSONNELS TRANSPARENTES ET PEU MOUSSANTE

(30) Priority: 30.06.2021 EP 21182747
(43) Date of publication of application: 08.05.2024
(73) Proprietor: Unilever IP Holdings B.V., 3013 AL Rotterdam (NL); Unilever Global IP Limited, Wirral, Merseyside CH62 4ZD (GB)
(72) Inventor: ASSIGHAOU, Souad, 6708 WH Wageningen (NL); DICKINSON, Kelvin, Brian, 6708 WH Wageningen (NL); KELSO, Hailey, 6708 WH Wageningen (NL); SIMON, Amelie, Laura, 6708 WH Wageningen (NL)
(74) Representative: Chisem, Janet
(86) International application number: PCT/EP2022/066058
(87) International publication number: WO 2023/274707

(56) References cited:
- US-A- 4 132 678
- US-A1- 2004 116 311
- US-A1- 2004 131 576
- US-A1- 2009 053 161
- US-A1- 2011 318 294
- US-A1- 2014 186 284

## Description

### Technical Field

This invention relates to a transparent conditioning composition, and to a method of treating hair with said composition.

### Background

Cosmetic and personal care compositions are often required to have multiple visual and functional attributes. For example, shampoo and hair conditioner compositions are generally required to have properties in addition to their ability to clean and/or condition hair in order to be appealing to the consumer.

Clear, transparent compositions are often desirable visually, but difficult to formulate. Additional benefit agents, such as silicones, cause undesirable effects such as clouding and discolouration.

Wet conditioning and dry conditioning are different benefits. Hair conditioners are required to deliver high levels of conditioning feel (slippery, smooth, lubricity, detangling) to wet hair. Foaming during use is strongly disliked by consumers.

Product rheology is an important attribute to consumers. Conditioners having superior rheology, such as thickness and yield stress can provide improved conditioning benefits and are preferred by consumers. Thin conditioner products are strongly disliked.

Most conditioners are effective because they contain a dispersed lamellar gel phase that is highly efficient at detangling. However, these are opaque because of the dispersed phase. Attempts have been made in the past to make the dispersed lamellar gel phase fragments small enough to make the composition transparent but processing and stability problems occur. Other known transparent conditioners are mainly polymer based and less effective at delivering conditioning benefits.

US 2014/186284 A (L'Oreal) discloses conditioning compositions for keratinous substrates. A clear conditioner formulation containing a quaternary material, PQ10, with a cationic surfactant is exemplified.

US 2004/131576 A (L'Oreal) discloses compositions comprising a quaternary silicone and a liquid fatty alcohol and method of treatment. Transparent compositions are envisaged and exemplified.

WO 00/06102 discloses a hair care cream composition comprising a cationic polymer of hydroxyethyl cellulose, cetrimonium chloride and ammonium lauryl sulphate. The composition is not transparent.

US2009/053161 discloses a process for managing hair, involving applying an aqueous composition comprising: (a) a compound chosen from a phospholipid, a polyamine, a fatty monoamine, and a fatty quaternary amine; (b) a nonionic surfactant; (c) a compound chosen from an alkyl(ether)carboxylate having 6 to 40 carbon atoms, and an alkyl(ether)phosphate having from 6 to 40 carbon atoms; (d) a water-insoluble material; and (e) a film former.

US2011/318294 discloses a method of imparting superior conditioning to hair comprising applying a composition comprising: (a) a polyquaternium compound having 100 or more quaternary nitrogen groups and having a weight average MWt of 30,000 to 1,000,000; (b) a nonalkoxylated phosphate ester of fatty alcohols wherein said phosphate esters have a fatty alcohol based group having a chain length of C₁₂ and C₁₈, at a ratio of said phosphate esters to said polyquaternium compound of at least 2:1 and wherein a total amount of said phosphate esters and said polyquaternium compounds range from 0.5 % and 5 % by weight of said personal care product, and (c) an additional ingredient, wherein said personal care product is capable of reducing a wet combing force of a hair sample treated therewith by at least about 10 Joules relative to water.

There remains a need for improved transparent cosmetic and personal care compositions, which can deliver a high level of conditioning benefits along with superior visual properties, whilst creating minimal foam during use.

We have surprisingly found that a transparent conditioner can be provided by use of a cationic polymer in combination with an ethoxylated anionic surfactant that comprises a high number of ethylene oxide groups, gives excellent visual attributes without compromising on conditioning performance and displaying minimal foam.

### Definition of the Invention

According to the present invention, in a first aspect, there is provided an aqueous conditioning composition comprising:
i) 0.1 to 2 wt % of a cationic conditioning polymer;
ii) 0.1 to 5 wt % of an anionic surfactant comprising EO groups, with a degree of ethoxylation of from 3 to 15;

wherein the composition has a transparency such that the turbidity is lower than 1 cm⁻¹, as measured using a UV/vis spectrophotometer and applying the equation Turbidity = (2.3 x A/L), where A is the absorbance measured from the sample at 750 nm and L is the path length; and
wherein the composition has a maximum foam height of 10 ml, as measured at 25°C and atmospheric pressure, by diluting 1g composition with 9 g water in a 100ml graduated cylinder with an internal diameter of 29 mm, and recording a starting volume (V1); the cylinder then being stoppered and vigorously shaken vertically for 10 seconds, followed by resting for 60 seconds after which measuring the height of the foam to the nearest 5ml graduation; and subtracting the starting volume (V1) from this value.

In a second aspect, the invention provides a method of treating hair, preferably wet hair, comprising applying to the hair a composition in accordance with the first aspect of the invention.

Also provided is a method of conditioning wet hair, preferably by reducing the coefficient of friction of the hair, comprising applying to the hair a composition of the first aspect of the invention.

A use of a composition of the first aspect of the invention to condition hair, preferably wet hair, preferably to reduce the coefficient of friction of the hair.

### General Description of the Invention

The compositions of the invention are transparent and low foaming.

The level of foaming produced by the compositions of the invention can be measured using any suitable method. A preferred method is a Cylinder Shake Test, as follows.

The cylinder shake test is preferably carried out under ambient conditions (25 °C and atmospheric pressure).

The test product (1 g) is diluted with water (9 g) and added to a 100 ml graduated cylinder. The starting volume (V1) is recorded. The cylinder is stoppered and then vigorously shaken vertically for 10 seconds. After a further 60 seconds rest, the height of the foam is measured visually to the nearest 5ml graduation. The starting volume (V1) of solution is subtracted from this value, the result of which indicates the amount of air entrained in the foam, or "foam height".

A suitable 100ml graduated glass cylinder is one manufactured by Duran, supplied by VWR and with an internal diameter of 29 mm.

By this method, the maximum height of foam produced by compositions of the invention is 10 ml, preferably 7 ml, more preferably 5 ml.

A suitable method of assessing transparency is to measure turbidity. UV-vis spectrometry may be used to determine the turbidity of the formulation. An example of a suitable spectrophotomer is a Jasco V-650 spectrophotometer.

Translucency (or turbidity) in a liquid product is due to suspended or colloidal particles that cause light to be scattered rather than transmitted in straight lines through the sample.

Turbidity may be calculated using the equation, where turbidity is 2.3 multiplied by absorbance divided by the path length of the sample, i.e. Turbidity = (2.3 x A/L); where A is the absorbance measured from the sample at 750 nm and L is the path length. Preferably a path length of 1.0 cm is used.

A composition is said to be transparent when the turbidity is lower than 1 cm⁻¹, preferably lower than 0.5 cm⁻¹, more preferably lower than 0.4 cm⁻¹, even more preferably lower than 0.25 cm⁻¹ most preferably lower than 0.1 cm⁻¹, as measured using a UV/visible spectrophotometer and applying the equation turbidity = (2.3*A/L), where A is the absorbance measured from the sample at 750 nm and L is the path length.

### The cationic conditioning polymer

The compositions of the invention comprise at least one cationic conditioning polymer.

The cationic conditioning polymer is present in an amount of 0.1 to 2 wt %, preferably 0.1 to 1 wt %, by total weight of the composition.

Preferably, the polymer has a polysaccharide backbone, wherein the polysaccharide comprises cationic modification. Preferably, the cationic modification comprises an amino group, for example a quaternary ammonium group.

Preferably the polysaccharide backbone is cellulosic. Most preferably the cellulosic backbone is hydroxy ethyl cellulose.

A preferred polymer is commercially available as Polyquaternium-10 (PQ10) for example UCare ^{™} polymer JR-30M from Dow.

### The ethoxylated anionic surfactant

The compositions of the invention comprise an ethoxylated anionic surfactant. The soluble ethoxylated anionic surfactant comprises ethylene oxide (EO) groups, with a degree of ethoxylation (n) of from 3 to 15, preferably from 5 to 15.

The ethoxylated anionic surfactant is preferably present in an amount of 0.1 to 5 wt %, preferably 0.1 to 2 wt %, by weight of the total composition.

Preferably the ethoxylated anionic surfactant is linear.

The anion is preferably a phosphate or a sulphate group.

Examples of preferred ethoxylated anionic surfactants are Oleth-10-Phosphate and PPG-5-Ceteth-10 Phosphate.

These are available from Croda as Crodafos ^{™} O10A, and Crodafos^{™} SG respectively.

### Cationic surfactant

The composition preferably comprises a cationic surfactant. The presence of a cationic surfactant aids transparency. This is particularly useful if other components, for example perfume, are added.

Preferred cationic surfactants may be selected from quaternary ammonium surfactants and tertiary ammonium surfactants.

Preferred cationic surfactants include coco-amine ethoxylates, for example Ethomeen^{™} C/25 or C/15, available from Nouryon. Also, PEG-15 Cocamine and PEG-2 Cocamine and cetyltrimethylammonium chloride cetyltrimethylammonium chloride (CTAC).

Compositions of the invention comprise cationic surfactants preferably comprising amino or quaternary ammonium hydrophilic moieties which are positively charged when dissolved in an aqueous composition.

Examples of suitable cationic surfactants correspond to the following general formula:

[N(R1)(R2)(R3)(R4)]+ (X)-

in which R1, R2, R3, and R4 are each independently selected from
   (a) an aliphatic group of from 1 to 22 carbon atoms, or
   (b) hydrogen or a polyoxyalkylene, hydroxyalkyl, aromatic, alkoxy, alkylamido, hydroxyalkyl, aryl, alkylaryl group having up to 22 carbon atoms; and
X is a salt-forming anion such as those selected from halide, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

Specific examples of such quaternary ammonium cationic surfactants of the above general formula are PEG-15 Cocamine and PEG-2 Cocamine, cetyltrimethylammonium chloride (CTAC) and salts of these, where the chloride is replaced by other halide (e.g., bromide), acetate, citrate, lactate, glycolate, phosphate nitrate, sulphate, or alkylsulphate.

In a preferred class of cationic surfactant of the above general formula, R1 is a C₂ to C₂₂ saturated or unsaturated, preferably saturated, alkyl chain and R², R³ and R⁴ are each independently selected from CH₃ and (CH₂CH₂O)nH, preferably (CH₂CH₂O)nH.

The level of cationic surfactant is preferably from 0.05 to 5, preferably 0.1 to 2 wt. % of the total composition.

A further acid used may be used to protonate the amine. Suitable acids include hydrochloric acid, citric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, lactic acid and mixtures thereof.

Mixtures of any of the above-described cationic surfactants may also be suitable.

### Rheology modifier

The compositions of the invention preferably comprise a rheology modifier to provide improved spreading of the composition on the hair. This thickens the composition.

Where present, preferably, the rheology modifier is present in an amount of from 0.2 to 2 wt %, most preferably from 0.5 to 1.5 wt %.

Preferably, the structurant is a polysaccharide, preferably derived from cellulose.

Preferably, the structurant is non-ionic.

Preferably, the structurant has a molecular weight ranging from 500 kDa to 2 MDa.

Examples of suitable structurants include Hydroxy Ethyl Cellulose (HEC) available, for example, under the tradename Natrosol, in a range available from Ashland. Another suitable example is Amaze XT, available from Nouryon.

Preferred rheology modifiers include hydroxy ethyl cellulose and hydroxy propyl methyl cellulose. The most preferred structurant is Hydroxy Ethyl Cellulose.

The compositions of the invention preferably have a Newtonian viscosity of from 0.001 to 5000 Pa.s, preferably from 0.1 to 1000 Pa s, more preferably from 1 to 500 Pa s, most preferably from 1 to 100 Pa.s when measured using a standard rheometer (e.g. DHR from TA instrument) with a cone and plate geometry at 25 °C.

### Solubiliser

The compositions may comprise a solubiliser to improve transparency.

A solubiliser is particularly useful to aid solubilisation of components such as perfumes.

Preferred solubilisers are non-ionic in character. They may be selected from nonionic surfactants and nonionic emulsifiers.

Preferred solubilisers are Polysorbate 20, for example Tween^{™}, available from Croda and Laureth-7, for example Marlipal 24/70 from Sasol.

The solubiliser, where present, is preferably present in an amount of from 0.1 to 5 wt %, more preferably from 0.2 to 2 wt %, most preferably from 0.25 to 1.5 wt %, by weight of the total composition.

The compositions of the invention comprise water. The compositions suitably comprise water in amount of from 60 to 98 wt % by weight of the total composition, preferably from 80 to 97 wt %, most preferably from 90 to 97 wt %.

The composition of the invention are preferably free from dispersed phases that affect the transparency (ie are not dissolved), for example silicone emulsion.

The pH of the compositions is preferably from 3 to 7, more preferably from 3.5 to 6.

### Further Ingredients

The composition according to the invention may comprise any of a number of ingredients which are common to hair compositions.

Other ingredients may include deposition polymers, preservatives, colouring agents, polyols such as glycerine and polypropylene glycol, chelating agents such as EDTA, antioxidants such as vitamin E acetate, fragrances, antimicrobials and pH adjusters for example acids, preferably organic acids. Each of these ingredients will be present in an amount effective to accomplish its purpose.

Preferably, the further ingredients include perfumes, preservatives and antimicrobials.

Mixtures of any of the above active ingredients may also be used.

Generally, such ingredients are included individually at a level of up to 5 wt %, preferably 2%, most preferably up to 1%, by weight of the total composition.

Preferably, the compositions of the invention are free from mineral oil. In the context of the invention, by free from is meant having less than 0.4 weight %, more preferably less than 0.1 weight %, even more preferably less than 0.05 weight %, still more preferably less than 0.001 weight %, yet preferably less than 0.0001 weight %, and most preferably 0 weight % of mineral oil by weight of the total composition.

Embodiments of the invention are given in the following examples, in which all percentages are quoted by weight based on total weight unless otherwise stated.

### Examples

The invention will now be illustrated by the following non-limiting examples. In the examples and throughout this specification, all percentages are by weight based on total composition unless indicated otherwise.

### Example 1 - preparation of Compositions 1-5 in accordance with the invention and Comparative Compositions A and B

The following compositions were prepared, where 1 - 5 are in accordance with the invention and A is a comparative. Comparative B was also prepared, as a composition representative of the prior art (Example 'I' from Example 1 of US20090053161A1):
Composition 1 comprises a cationic conditioning polymer (Polyquaternium-10) and an ethoxylated anionic surfactant comprising 10 EO groups (Oleth-10-Phosphate).
Compositions 2-5 comprise further optional ingredients.

Composition A comprises sodium lauryl ether phosphate (SLES) 1EO in place of oleth-10.

Composition B comprises Behentrimonium Chloride (BTAC) (1 wt %), Procetyl AWS (PPG-5-Ceteth-20) (25 wt %), Crodafos NIO Acid (Oleth-10 phosphate) (1 wt %), Mineral oil (2 wt %) and Resyn 2829-30, neutralised with AMP (at 3 wt %).

Where a perfume is present, cationic surfactant and a polysorbate-20 are present in order to solubilise the perfume and maintain transparency.
- Composition 1 - no cationic surfactant, polysorbate, fragrance or HEC
- Composition 2 - same as 1 but with HEC
- Composition 3 - same as 2 with cationic surfactant
- Composition 4 - contains perfume, solubiliser and cationic surfactant
- Composition 5 - contains perfume, solubiliser, cationic surfactant & HEC
- Comparative Composition A, same as 5 but with oleth-10 (10 EO) replaced by sodium lauryl ether phosphate (SLES) 1 EO
- Comparative Composition B, from the prior art

The commercially available shampoo, "Dove Intensive Repair Shampoo" was used as a comparative in foam generation tests. This product typically contains about 12 wt % SLES.

The compositions of Compositions 1 - 5 and A are given in Table 1 below:

**Table 1: Compositions of Compositions 1 - 5, in accordance with the invention and Comparative Composition A**

| Ingredients INCI | Amount (wt %) | | | | | |
|---|---|---|---|---|---|---|
| | 1 | 2 | 3 | 4 | 5 | A |
| PEG-15 Cocamine | - | - | - | 1 | 1 | 1 |
| PEG-2 Cocamine | - | - | 0.75 | - | - | - |
| Hydroxyethylcellulose | - | 1 | 1 | - | 1 | 1 |
| Polyquaternium-10 | 0.5 | 0.5 | 0.5 | 0.5 | 0.5 | 0.5 |
| Polysorbate 20 | - | - | - | 1 | 1 | 1 |
| Oleth-10-Phosphate | 1 | 1 | 1 | 1 | 1 | - |
| Sodium laureth sulfate | - | - | - | - | - | 1 |
| Parfum | - | - | - | 0.8 | 0.8 | 0.8 |
| Citric Acid | to pH 4-5 | to pH 4-5 | to pH 4-5 | to pH 4-5 | to pH 4-5 | to pH 4-5 |
| Water and minors (preservatives, etc) | to 100% | to 100% | to 100% | to 100% | to 100% | to 100% |

The compositions in table 1 were prepared as follows:
1. Water (300 ml) was provided in a first vessel at 25°C.
2. Where present, hydroxyethylcellulose was added to the water, with stirring for 2 minutes.
3. The cationic conditioning polymer (Polyquaternium-10) was added and the mixture stirred for a further 2 minutes.
4. Where present, cationic surfactant (PEG-15 cocamine or PEG-2 Cocamine) was added and the mixture stirred for a further 2 minutes.
5. In a second vessel, anionic surfactant was added along with any minor ingredients to water (35 ml) at 70 °C and gently stirred until dissolved.
6. The resulting hot solution was then added to the mixture in the first vessel, which was heated to 40°C with stirring at 100rpm for 30 minutes before being cooled to room temperature. Finally, any remaining ingredients (fragrance, polysorbate 20, citric acid) were added with continued stirring at 100rpm.

The composition of Composition of Comparative B is given in Table 2 below:

**Table 2: Composition Comparative Composition B**

| Ingredients (INCI) | Amount (wt %) |
|---|---|
| Behentrimonium Chloride (BTAC) | 1 |
| Procetyl AWS (PPG-5-Ceteth-20) | 25 |
| Crodafos NIO Acid (Oleth-10 phosphate) | 1 |
| Mineral oil | 2 |
| Resyn 2829-30, neutralised with AMP (vinyl acetate/crotonates/vinyl neodecanoate copolymer) | 5 |
| Adjusted to 100 % with water | To 100 |

The compositions in table 1 were prepared as follows:
1. Cationic surfactant was added to a suitable vessel and hydrated with hot water (70°C)
2. The anionic surfactant was then added with mixing
3. The mineral oil was then added with mixing
4. Neutralised Resyn 28290-30 polymer was then added and mixed
5. In a second vessel, Crodafos NIO Acid was added to water (35 ml) at 70 °C and gently stirred until dissolved.

All processing was carried out using low shear (with an overhead stirrer).

The additions of anionic surfactant, mineral oil, styling polymer solution, and nonionic surfactant were made whilst the solution was cooling.

### Example 2: Transparencies of Compositions 1, 2, 4 & 5, in accordance with the invention and Comparative Composition A

The transparencies of conditioner compositions 1, 2, 4 & 5 and A were evaluated by measuring the turbidity. The conditioner formulation is said to be transparent when the turbidity is lower than 1.0 cm⁻¹.

The transparency of each formulation was measured using a Jasco V-650 spectrophotometer. Absorbance at 750nm was converted into a turbidity value according to the equation: Turbidity = (2.3 x Absorbance)/path length, where A is the absorbance measured from the sample at 750 nm and the path length, which was 1.0 cm.

Turbidities of Compositions 1, 2, 4 & 5 and A are given in Table 3 below.

**Table 3: Turbidities of Compositions 1, 2, 4 & 5 and A**

| Example | Turbidity (cm⁻¹) |
|---|---|
| 1 | 0.09 |
| 2 | 0.36 |
| 4 | 0.23 |
| 5 | 0.01 |
| A | 0.40 |

A transparent sample has a turbidity below 1.0 cm⁻¹.

Whilst A presents as transparent, it is a high foaming composition (Table 3 below)

### Example 3: Foaming properties of Compositions 1, 2, 4 & 5, in accordance with the invention and Comparative Composition A

Consumers do not accept foaming from a hair conditioner during use. Foam is strongly associated with cleansing and not with caring.

The foaming levels of conditioner compositions 1, 2, 4 & 5 and A were evaluated.

A commercially available shampoo, "Dove Intensive Repair Shampoo", was also used as a comparison.

### Cylinder Shake Foam Test

The level of foaming produced by the compositions of the invention was measured using the following method at ambient temperature (25°C) and atmospheric pressure.

1g of the test product was diluted with 9g of water and added to a 100 ml graduated glass cylinder, manufactured by Duran, supplied by VWR, and with an internal diameter of 29 mm. The starting volume (V1) was recorded. The cylinder was stoppered and then vigorously shaken vertically for 10 seconds. After a further 60 seconds rest, the height of the foam was measured visually to the nearest 5ml graduation. The starting volume of solution (V1 = 10ml) was subtracted from this value to calculate the amount of air entrained in the foam.

The results are shown in Table 4.

**Table 4: Foam height for compositions compositions 1, 2, 4 & 5 and A; as well as a commercially available shampoo**

| Composition Description | Height of Foam; n=5 | |
|---|---|---|
| | Average /ml | Standard Deviation /ml |
| 1 | 5 | 0 |
| 2 | 5 | 0 |
| 4 | 5 | 0 |
| 5 | 5 | 0 |
| A | 15 | 0 |
| Dove Intensive Repair Shampoo | 78 | 3 |

It will be seen that the compositions of the invention exhibit very low levels of foam. For comparative example A, even at only 1 wt % of SLES-1 EO significantly more foam is created than when the highly ethoxylated phosphate ester is used.

### Example 4: Conditioning properties of compositions 2, 4 & 5 and A

The level of friction of hair may be used as an indication of the level of lubricity imparted by a conditioning treatment.

Coefficient of friction is a dimensionless number that is defined as the ratio between friction force and normal force.

Hair (5g hair switch) was first wetted with water and treated with 1g of composition (2, 4 & 5 and A) before being diluted with a further 7ml of water.

Coefficents of friction (CoF) were then determined by stroking a hair switch with a real finger on a bespoke instrumented force plate which used 6 force transducers to measure forces and torques in three dimensions.

A commercially available transparent conditioner, "L'Oreal Fibrology Transparent Conditioner", was also used as a comparison.

The results are given in Table 5.

**Table 5: Coefficient of friction of hair treated with compositions 2, 4 & 5 and A; as well as a commercially available conditioner**

| Composition | CoF after 7ml water added |
|---|---|
| 2 | 0.3345 |
| 4 | 0.3238 |
| 5 | 0.3276 |
| L'Oreal Fibrology Transparent Conditioner | 0.4095 |

It can be seen that the compositions in accordance with the invention provide low friction to hair.

### Example 5: Conditioning properties of comparative composition B against composition 5

The level of friction of hair treated with Comparative Composition B, representative of the prior art, was measured in the same way as described above. The results are given in the Table below:

**Table 6: Coefficient of friction of hair treated with comparative example B and composition 5**

| Composition | CoF after 7ml water added |
|---|---|
| 5 | 0.3276 |
| B | 0.6039 |

It will be seen that the composition 5 in accordance with the invention provides a dramatically lower friction to wet hair than the composition of the prior art.

In addition, the reworked example was observed to be very thick in consistency and well outside the preferred viscosity limit of the invention. Further, the composition was non-transparent in appearance.

## Claims

1. An aqueous conditioning composition comprising:
i) 0.1 to 2 wt % of a cationic conditioning polymer;
ii) 0.1 to 5 wt % of an anionic surfactant comprising EO groups, with a degree of ethoxylation of from 3 to 15;
wherein the composition has a transparency such that the turbidity is lower than 1 cm⁻¹, as measured using a UV/vis spectrophotometer and applying the equation Turbidity = (2.3 x A/L), where A is the absorbance measured from the sample at 750 nm and L is the path length; and
wherein the composition has a maximum foam height of 10 ml, as measured at 25°C and atmospheric pressure, by diluting 1g composition with 9 g water in a 100ml graduated cylinder with an internal diameter of 29 mm, and recording a starting volume (V1); the cylinder then being stoppered and vigorously shaken vertically for 10 seconds, followed by resting for 60 seconds after which measuring the height of the foam to the nearest 5ml graduation; and subtracting the starting volume (V1) from this value.

2. A composition as claimed in claim 1, which further comprises a cationic surfactant.

3. A composition as claimed in claim 2, wherein the level of cationic surfactant is from 0.05 to 5 wt %, preferably 0.1 to 2 wt % of the total composition.

4. A composition as claimed in claim 2 or claim 3, wherein the cationic surfactant is selected from a quaternary ammonium surfactant and a tertiary ammonium surfactant.

5. A composition as claimed in any preceding claim, wherein the cationic conditioning polymer is present in an amount of from 0.1 to 1 wt %.

6. A composition as claimed in any preceding claim, wherein the cationic conditioning polymer has a polysaccharide backbone wherein the polysaccharide comprises cationic modification.

7. A composition as claimed in claim 6, wherein the cationic modification comprises an amino group.

8. A composition as claimed in any preceding claim, wherein the ethoxylated anionic surfactant comprises ethylene oxide groups, with a degree of ethoxylation, n, of from 5 to 15.

9. A composition as claimed in any preceding claim, wherein the ethoxylated anionic surfactant is present in an amount of from 0.1 to 2 wt %.

10. A composition as claimed in any preceding claim, which further comprises a rheology modifier in an amount of from 0.2 to 2 wt %.

11. A composition as claimed in any preceding claim, which further comprises a solubiliser.

12. A composition as claimed in claim 11, wherein the solubiliser is present in an amount of from 0.1 to 5 wt %, more preferably from 0.2 to 2 wt %, most preferably from 0.25 to 1.5 wt %.

13. A composition as claimed in any preceding claim, having a Newtonian viscosity of from 0.001 to 5000 Pa.s, preferably from 0.1 to 1000 Pa s, more preferably from 1 to 500 Pa s, most preferably from 1 to 100 Pa.s when measured using a standard rheometer (e.g. DHR from TA instrument) with a cone and plate geometry at 25 °C.

14. A method of treating wet hair, comprising the step of applying to the hair a composition as defined in any of claims 1 to 13.

15. A use of a composition as defined in claims 1 to 13 to condition hair, preferably wet hair.

## Patentansprüche

1. Wässrige konditionierende Zusammensetzung, umfassend:
i) 0,1 bis 2 Gew.-% eines kationischen konditionierenden Polymers;
ii) 0,1 bis 5 Gew.-% eines anionischen Tensids, umfassend EO-Gruppen mit einem Ethoxylierungsgrad von 3 bis 15;
wobei die Zusammensetzung eine solche Transparenz aufweist, dass die Trübung weniger als 1 cm⁻¹ beträgt, gemessen mit einem UV/VIS-Spektralphotometer und unter Anwendung der Gleichung Trübung = (2,3 x A/L), wobei A die bei 750 nm von der Probe gemessene Absorption und L die Weglänge ist; und
wobei die Zusammensetzung eine maximale Schaumhöhe von 10 ml aufweist, gemessen bei 25°C und Atmosphärendruck, indem 1 g Zusammensetzung mit 9 g Wasser in einem 100 ml-Messzylinder mit einem Innendurchmesser von 29 mm verdünnt und ein Ausgangsvolumen (V1) aufgezeichnet wird, wobei der Zylinder dann wieder verschlossen und 10 Sekunden lang kräftig vertikal geschüttelt wird, gefolgt von einer Ruhezeit von 60 Sekunden, wonach die Höhe des Schaums auf 5 ml genau gemessen und das Ausgangsvolumen (V1) von diesem Wert abgezogen wird.

2. Zusammensetzung, wie im Anspruch 1 beansprucht, die außerdem ein kationisches Tensid umfasst.

3. Zusammensetzung, wie im Anspruch 2 beansprucht, wobei der Anteil des kationischen Tensids 0,05 bis 5 Gew.-%, bevorzugt 0,1 bis 2 Gew.-% der gesamten Zusammensetzung beträgt.

4. Zusammensetzung, wie im Anspruch 2 oder Anspruch 3 beansprucht, wobei das kationische Tensid unter einem quaternären Ammoniumtensid und einem tertiären Ammoniumtensid ausgewählt ist.

5. Zusammensetzung, wie in einem vorhergehenden Anspruch beansprucht, wobei das kationische konditionierende Polymer in einer Menge von 0,1 bis 1 Gew.-% vorliegt.

6. Zusammensetzung, wie in einem vorhergehenden Anspruch beansprucht, wobei das kationische konditionierende Polymer ein Polysaccharid-Grundgerüst aufweist, wobei das Polysaccharid eine kationische Modifikation umfasst.

7. Zusammensetzung, wie im Anspruch 6 beansprucht, wobei die kationische Modifikation eine Aminogruppe umfasst.

8. Zusammensetzung, wie in einem vorhergehenden Anspruch beansprucht, wobei das ethoxylierte anionische Tensid Ethylenoxidgruppen mit einem Ethoxylierungsgrad n von 5 bis 15 umfasst.

9. Zusammensetzung, wie in einem vorhergehenden Anspruch beansprucht, wobei das ethoxylierte anionische Tensid in einer Menge von 0,1 bis 2 Gew.-% vorliegt.

10. Zusammensetzung, wie in einem vorhergehenden Anspruch beansprucht, die außerdem ein Rheologie-Modifizierungsmittel in einer Menge von 0,2 bis 2 Gew.-% umfasst.

11. Zusammensetzung, wie in einem vorhergehenden Anspruch beansprucht, die außerdem einen Lösungsvermittler umfasst.

12. Zusammensetzung, wie im Anspruch 11 beansprucht, wobei der Lösungsvermittler in einer Menge von 0,1 bis 5 Gew.-%, bevorzugter von 0,2 bis 2 Gew.-%, höchst bevorzugt von 0,25 bis 1,5 Gew.-%, vorliegt.

13. Zusammensetzung, wie in einem vorhergehenden Anspruch beansprucht, die eine Newtonsche Viskosität von 0,001 bis 5000 Pa.s, bevorzugt von 0,1 bis 1000 Pa.s, bevorzugter von 1 bis 500 Pa.s, höchst bevorzugt von 1 bis 100 Pa.s, aufweist, wenn unter Verwendung eines Standardrheometers (z.B. DHR von TA Instrument) mit einer Kegel- und Plattengeometrie bei 25°C gemessen wird.

14. Verfahren zur Behandlung von nassem Haar, umfassend den Schritt des Auftragens einer Zusammensetzung, wie in einem der Ansprüche 1 bis 13 beansprucht, auf das Haar.

15. Verwendung einer Zusammensetzung, wie in den Ansprüchen 1 bis 13 definiert, um Haar zu konditionieren, bevorzugt nasses Haar.

## Revendications

1. Composition de conditionnement aqueuse comprenant:
i) 0,1 à 2 % en poids d'un polymère de conditionnement cationique;
ii) 0,1 à 5 % en poids d'un tensioactif anionique comprenant des groupes OE, avec un degré d'éthoxylation de 3 à 15;
où la composition a une transparence telle que la turbidité est inférieure à 1 cm⁻¹, telle que mesurée à l'aide d'un spectrophotomètre UV/Vis et en appliquant l'équation Turbidité = (2,3 x A/L), où A est l'absorbance mesurée à partir de l'échantillon à 750 nm et L est le trajet optique; et
où la composition a une hauteur de mousse maximale de 10 ml, telle que mesurée à 25°C et à pression atmosphérique, en diluant 1 g de composition avec 9 g d'eau dans une éprouvette graduée de 100 ml avec un diamètre interne de 29 mm, et en enregistrant un volume de départ (V1); l'éprouvette étant ensuite bouchée et agitée vigoureusement verticalement pendant 10 secondes, puis mise à reposer pendant 60 secondes, après quoi la hauteur de la mousse est mesurée jusqu'à la graduation de 5 ml la plus proche; et le volume de départ (V1) est soustrait de cette valeur.

2. Composition selon la revendication 1, qui comprend en outre un tensioactif cationique.

3. Composition selon la revendication 2, où le taux de tensioactif cationique est de 0,05 à 5 % en poids, de préférence de 0,1 à 2 % en poids de la composition totale.

4. Composition selon la revendication 2 ou la revendication 3, où le tensioactif cationique est choisi parmi un tensioactif d'ammonium quaternaire et un tensioactif d'ammonium tertiaire.

5. Composition selon l'une quelconque des revendications précédentes, où le polymère de conditionnement cationique est présent en une quantité de 0,1 à 1 % en poids.

6. Composition selon l'une quelconque des revendications précédentes, où le polymère de conditionnement cationique a un squelette de polysaccharide dans lequel le polysaccharide comprend une modification cationique.

7. Composition selon la revendication 6, où la modification cationique comprend un groupe amino.

8. Composition selon l'une quelconque des revendications précédentes, où le tensioactif anionique éthoxylé comprend des groupes oxyde d'éthylène, avec un degré d'éthoxylation, n, de 5 à 15.

9. Composition selon l'une quelconque des revendications précédentes, où le tensioactif anionique éthoxylé est présent en une quantité de 0,1 à 2 % en poids.

10. Composition selon l'une quelconque des revendications précédentes, qui comprend en outre un modificateur de rhéologie en une quantité de 0,2 à 2 % en poids.

11. Composition selon l'une quelconque des revendications précédentes, qui comprend en outre un solubilisant.

12. Composition selon la revendication 11, où le solubilisant est présent en une quantité de 0,1 à 5 % en poids, de préférence encore de 0,2 à 2 % en poids, de manière particulièrement préférable de 0,25 à 1,5 % en poids.

13. Composition selon l'une quelconque des revendications précédentes, ayant une viscosité newtonienne de 0,001 à 5000 Pa.s, de préférence de 0,1 à 1000 Pa.s, de préférence encore de 1 à 500 Pa.s, de manière particulièrement préférable de 1 à 100 Pa.s lorsqu'elle est mesurée à l'aide d'un rhéomètre standard (par exemple DHR de TA instrument) avec une géométrie cône et plaque à 25°C.

14. Procédé de traitement des cheveux mouillés, comprenant l'étape d'application aux cheveux d'une composition telle que définie dans l'une quelconque des revendications 1 à 13.

15. Utilisation d'une composition telle que définie dans les revendications 1 à 13 pour conditionner les cheveux, de préférence les cheveux mouillés.
